(19) Europäisches Patentamt

European Patent Office

Office européen des brevets

(11) **EP 3 456 249 A1**

(12) **EUROPEAN PATENT APPLICATION**

(43) Date of publication:
20.03.2019   Bulletin 2019/12

(51) Int Cl.:
*A61B 5/02* (2006.01)          *G16H 30/20* (2018.01)
*G16H 10/60* (2018.01)        *A61B 5/0215* (2006.01)
*A61B 5/00* (2006.01)

(21) Application number: 17191426.0

(22) Date of filing: 15.09.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **HAASE, Christian**
  **5656 AE Eindhoven (NL)**
• **GRASS, Michael**
  **5656 AE Eindhoven (NL)**
• **VAN LAVIEREN, Martijn Anne**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **USING ARCHIVED PATIENT DATA TO CORRECT INTRAVASCULAR MEASUREMENTS FOR PATIENT CO-MORBIDITIES**

(57)    An apparatus is provided which improves the evaluation of a patient's vasculature by applying a correction to invasively acquired intravascular measurement data of a vessel of interest on the basis of archived patient data of said patient from a patient database. By correcting the measurement data, co-morbidities of the patient which may influence the intravascular measurement results are accounted for.

FIG. 1

EP 3 456 249 A1

**Description**

FIELD OF THE INVENTION

The present invention relates to an apparatus for evaluating a patient's vasculature, a corresponding system, a method and a respective computer program. In particular, the present invention relates to an improved evaluation of the patient's coronary vasculature, using an intravascular measurement of a vessel of interest and correcting the intravascular measurement results by means of historic patient data of the patient that has been archived in a respective patient database.

BACKGROUND OF THE INVENTION

Functional stenosis in coronary arteries is typically graded using Fractional Flow Reserve (FFR) or Instantaneous Wave-Free Ratio (iFR) as a surrogate for intravascular flow measurements. Both, FFR as well as iFR, are a measure for the pressure drop of the blood along the stenosis, i.e. are determined as the ratio of the pressure distal the stenosis ($P_d$) to the pressure in the aorta ($P_a$).

In these measurements, it is assumed that this pressure ratio $P_d/P_a$ is proportional to the relation $Q_{stenosis}/Q_{healthy}$. Hereby, $Q_{stenosis}$ is defined as the maximal volumetric blood flow rate in a vessel of interest affected by stenosis and $Q_{healthy}$ is defined as the maximal volumetric blood flow rate in the respective vessel of interest, if the vessel were healthy. Thus, it is assumed that the pressure ratio may be used as a measure for the remaining maximum flow capability through a vessel of interest affected by the stenosis.

While, thus far, the flow capabilities of a vessel of interest have been estimated using the above approximation $Q_{stenosis}/Q_{healthy} \approx P_d/P_a$, this approximation only holds true in case no further effects occur that influence and/or change the hemodynamics inside the vessel of interest. In that case, conclusions regarding the remaining maximum flow capabilities within the vessel of interest, drawn based on FFR/iFR measurements, may not be correct. Accordingly, this may result in an inaccurate evaluation of the patient's vasculature, for example leading to situations in which it is incorrectly determined that the patient does not have to be treated while, in reality, a treatment should be performed.

Given the importance of assessing treatment options correctly, an apparatus and a method is needed which enables a more accurate evaluation of the patient's vasculature.

SUMMARY OF THE INVENTION

It is therefore an object of the invention to provide a more accurate evaluation of the patient's vasculature. More particularly, it is an object of the invention to provide an improved apparatus for evaluating a patient's vasculature on the basis of an intravascular measurement which takes into account additional factors such as the patient's health status and/or the patient's co-morbidities when assessing the blood flow capabilities.

Thus, an apparatus for evaluating a patient's vasculature is provided, which comprises an input unit configured to receive intravascular measurement data acquired in-situ for a vessel of interest in the patient's vasculature. The input unit is further configured to receive archived patient data for the patient from a patient database. Further, the apparatus comprises a correction unit configured to apply a correction to the intravascular measurement data on the basis of archived patient data.

In this context, the term intravascular measurement data relates to data that has been acquired in-situ, i.e. from inside a vessel of interest in a patient's vasculature. More particularly, the term intravascular measurement data may refer to an intravascular measurement of one or more health parameters, in particular one or more hemodynamic parameters, inside the vessel of interest.

These measurements may typically be performed by introducing a catheter including a measurement device into the vessel of interest and using the measurement device to collect one or more respective health parameter values for the health parameter to be regarded. In that respect, the term vessel of interest may particularly refer to a vessel or a vessel segment in the patient's vasculature in which a stenosis is suspected.

The intravascular measurement data may particularly comprise a health parameter value or a plurality of health parameter values collected at a plurality of intravascular positions along a longitudinal axis of the vessel of interest.

According to a specific embodiment, the health parameters may particularly relate to a hemodynamic parameter, such as the blood pressure in the vessel of interest. Hereby, one or more blood pressure values may be collected at one or more intravascular positions inside the vessel of interest.

The term patient database refers to any kind of database in which patient data may be stored. The database may hereby be implemented by a server having one or more storage components, such as solid state memory devices, to store the archived patient data. The database may also be implemented as a server system including multiple servers, each having one or more respective storage components. The database may likewise be implemented as a storage component, such as a memory device, within a computer system. Likewise, the database may be implemented as a single storage component in a computer system.

The archived patient data may be retrieved by accessing the database. In this context, the accessing may be performed automatically by the apparatus in response to the apparatus receiving the intravascular measurement data. Likewise, the apparatus may be triggered to access the database through a user input. Such user input may particularly relate to a request for evaluation of the patient's vasculature. Alternatively or additionally, the user input may relate to a user manually providing the intravascular measurement information. Further, the accessing the database may be performed manually by a user through a respective user terminal. That is, the user may actively seek to access the archived patient data from the database by inputting a respective request into a user terminal.

To this end, the apparatus, the database and/or the user terminal may be connected by a network connection. Hereby, the apparatus, the database and/or the user terminal may particularly be connected via the internet. In one embodiment, the database may be provided as a remote entity, such as a remote server or a remote server system. The apparatus and/or the user terminal may particularly be connected to the (remote) database via a cloud service and the accessing to and retrieval from the database may be performed via said cloud service.

Having the apparatus and/or the user terminal connected to the database via the internet and/or via a cloud service allows the database to be provided at a different location than the apparatus, thereby enabling a centralized storage of the archived patient data. This centralized storage may allow for the archived patient data to be accessed from different, spatially distributed treatment sites.

Alternatively or additionally, the apparatus, the database and/or the user terminal may be provided in the same local area network. They may hereby be connected to a network switch, such as a router, through a wireless or wired connection in said network. In an embodiment, it may also be possible to directly connect the apparatus, the database and/or the user terminal to one another, for example via a Bluetooth connection.

The term archived patient data refers to one or more additional patient information that has been obtained about the patient and stored in the patient database. According to an embodiment, the archived patient data may comprise personal patient information, such as age, gender, known diseases running in the patient's family or the like. The archived patient data may also comprise information about the patient's medical history, such as known chronic diseases, previous treatments, long-term medication or similar information. The archived patient data may further comprise historical medical measurement data, such as electrocardiographic (ECG-) data, diagnostic image data, or the like that has been acquired for the patient in the past. Likewise, the archived patient data may comprise the corresponding current medical measurement data of the patient, i.e. the corresponding medical data that has been recently acquired for the patient. As such, the archived patient data may particularly be suited to derive which co-morbidities (aside from the stenosis) the patient may suffer from.

In this context, the term diagnostic image data particularly refers to data that has been acquired using a diagnostic imaging modality. More particularly, the diagnostic image data may refer to image data acquired using X-ray scanning, computed tomography (CT), X-ray angiography, positron emission tomography (PET), single photon emission computed tomography (SPECT), ultrasound imaging, or the like. The diagnostic image data may also be perfusion data obtained using magnetic resonance imaging (MRI), CT and/or PET. Further diagnostic image data that may be useful for the above objective may also be obtained.

Since the archived patient data provides further information about the patient's health state and/or existing co-morbidities, the archived patient data enables to determine that the intravascular measurement data may be influenced by irregularities and that certain health parameters used for evaluation thereof need to be corrected accordingly. To that end, the archived patient data may particularly be used to perform the necessary correction of one or more of the health parameters used for assessing the intravascular measurement data.

More specifically, the archived patient data may allow to determine the factors influencing the intravascular measurement data, more particularly influencing the health parameters used for assessment thereof. These factors may lead to a deviation from respective reference values for these health parameters. As an example, the archived patient data may indicate that the patient is suffering from a structural heart disease and/or from dysfunctions in the microvasculature. These co-morbidities may particularly influence the hemodynamic health parameter values which therefore differ from the corresponding reference values typically used to evaluate the patient's vasculature on the basis of the intravascular measurement data.

On the basis of the knowledge about these co-morbidities, the intravascular measurement data may thus be corrected accordingly. In the particular example, the structural heart disease may cause a hemodynamic health parameter value to be reduced compared to the reference value that would be considered in patients not suffering from co-morbidities, in this example in a patient not having the structural heart disease. Accordingly, when evaluating the patient's vasculature on the basis of the intravascular measurement data, a reduced hemodynamic health parameter value may be considered instead of the reference value that would be used in case no co-morbidities are affecting said health parameter. Taking into account this reduced hemodynamic health parameter may hereby improve the accuracy of the evaluation.

Thus, the improved apparatus allows to derive, from the archived patient data, one or more health parameters indicative of the patients health state. Using the further information, the apparatus is enabled to more accurately assess the intravascular measurement data acquired for the vessel of interest. In one particular example, the improved apparatus may allow to more accurately approximate the flow capabilities of the blood through the vessel of interest. For example, irregularities in the hemodynamics caused by changes of the blood properties, the vessel wall properties or the like may be compensated for.

According to a further embodiment, the intravascular measurement data is acquired by a measurement of a Fractional Flow Reserve (FFR) and/or an Instantaneous wave-Free Ratio (iFR).

In some embodiments, the intravascular measurement data may particularly correspond to a measurement of the Fractional Flow Reserve (FFR-) or the Instantaneous wave-Free Ratio (iFR-) value in a vessel of interest in the patient's vasculature.

FFR as well as iFR both relate to an invasive method for functional stenosis assessment. Particularly, an FFR/iFR acquisition is typically performed using a pressure wire as the intravascular measurement device. The pressure wire is placed distally to the stenosis to measure at least one distal pressure value $P_d$ while at the same time the guide catheter, e.g. in the coronary ostium, is used to record at least one aortic pressure value $P_a$. To make sure the two measurement systems are measuring with the same scale, a normalization at the same measurement position before the FFR/iFR acquisition is performed.

During the FFR/iFR acquisition the pressure wire may further be pulled back from the distal to a proximal position inside the vessel to record a multitude of pressure and FFR/iFR values along the vessel.

During an FFR measurement, the ratio $P_d/P_a$ is determined using whole-cardiac cycle pressure measurements. Thus, the pressure values to calculate this ratio have to be acquired during maximal hyperemic blood flow, such that the variability in the autoregulation of the microvascular resistance may be neglected. Accordingly, the patient has to be brought into a state of hyperemia during FFR measurements. This is typically achieved by the administration of vasodilatory agents.

In contrast, iFR measurements are performed at rest during a specific period of the cardiac cycle, namely the late diastole, which is also referred to as the wave-free period. During this period, the waves that affect coronary flow are dormant, which means that pressure and flow are linearly related and microvascular resistance is minimized. This enables an assessment of the ratio $P_d/P_a$ without the necessity of increasing the blood flow by means of vasodilatory agents.

FFR/iFR measurement values relate to health parameter values that are typically used as indices to indicate the remaining maximum flow capability through a vessel of interest using the above-mentioned approximation $Q_{stenosis}/Q_{healthy} \approx P_d/P_a$.

This approximation is derived based on the equation:

$$\frac{Q_{stenosis}}{Q_{healthy}} = \frac{\frac{1}{R_{M-stenosis}}(P_d - P_v)}{\frac{1}{R_{M-healthy}}(P_{a-healthy} - P_v)} \approx \frac{P_d}{P_a}.$$

Herein, $P_v$ is the venous pressure and $R_M$ is the resistance of the microvasculature. In order to arrive at the above approximation, it is assumed that the venous pressure may be neglected:

$$P_V \approx 0,$$

and that the resistance of the microvasculature is equal in both, the stenosis and the healthy, case:

$$R_{M-healthy} = R_{M-stenosis}.$$

As may be appreciated from the above equation, the factors influencing the relation between the remaining maximum flow capability and the pressure ratio are well-defined. This allows a convenient determination of the health parameters affect by and the corrections necessary in case of additional maladies.

As an example, it is assumed that the archived patient data indicates that the patient is suffering from a microvascular dysfunction. Since said microvascular dysfunction may particularly affect the resistance of the microvasculature, e.g. by increasing it, it may be derived from the archived patient data that the resistance of the microvasculature in the above equation has to be increased, compared to the reference value, for the stenosis-state.

Alternatively or additionally, the microvascular dysfunction may also reduce or increase the aortic pressure in the vessel of interest. Accordingly, it may be derived from the archived patient data, that the reference value ($P_{a-healthy}$) supposed to be used in the above equation has to be replaced by an aortic pressure value ($P_{a-diseased}$) which has a respectively reduced or increased value compared to the reference value.

To that end, in some embodiments, the position of the FFR/iFR measurement may be comprised in the patient data. This position may also be used as an indicator to determine a respective correction of the FFR/iFR measurement. More particularly, it may be determined that the lesion is at a proximal position. In this case, the lesion is of higher severity, since more myocardial mass is affected by it. This may indicate a correction of the FFR/iFR measurement that decreases the FFR/iFR value.

As such, using FFR/iFR measurements to acquire the intravascular measurement data allows for a more straight-forward determination of the health parameters affected by the co-morbidities indicated by the archived patient data and the corresponding corrections necessary.

According to a further embodiment, the apparatus further comprises an extraction unit configured to extract at least one correction factor based on the archived patient data. Hereby, the correction unit is configured to apply the at least one correction factor to the intravascular measurement data. In an even further embodiment, the at least one correction factor is related to an aortic pressure value and/or a resistance of a microvasculature of the patient.

In one embodiment, the archived patient data retrieved from the patient database may allow to determine which health parameters that are used when evaluating the patient's vasculature may be affected by co-morbidities of the patient, i.e. for which health parameters the reference values may be different in the diseased state.

In some embodiments, it may be derived from the archived patient data that the patient in question is suffering from a disease that causes a change in at least one hemodynamic health parameter of the patient, such as the blood viscosity, the blood pressure or the like. In that context, the archived patient data may particularly contain patient information concerning the respective measured hemodynamic health parameter values that have been acquired for the patient in previous examination sessions.

Using these measured hemodynamic health parameter values as archived in the database it is possible to determine the relationship between the hemodynamic health parameter values for the patient and the respective reference values. On the basis of this relationship, the correction factors may be derived for the hemodynamic health parameter showing a deviation from the reference for said health parameter. These correction factors may then be used in the evaluation of the intravascular measurement data to increase the accuracy of the intravascular measurement of the vessel of interest.

In order to further illustrate this concept, the above-described example is used once again. According to the example, the health parameters affected by co-morbidities of the patient correspond to the aortic pressure and the microvascular resistance. Hereby, the reference values for the aortic pressure $P_a$ and the microvascular resistance $R_M$ may be designated $P_{a\text{-}healthy}$ and $R_{M\text{-}healthy}$, respectively. Correspondingly, the aortic pressure and the microvascular resistance for the patient affected by co-morbidities may be designated $P_{a\text{-}diseased}$ and $R_{M\text{-}diseased}$, respectively.

In these cases, the relation between the reference values and the health parameter values for the patient suffering from co-morbidities may each be described by a respective correction factor according to:

$$\alpha\, P_{a-healthy} = P_{a-diseased},$$

$$\beta\, R_{M-healthy} = R_{M-diseased}.$$

As may be appreciated, the aortic pressure correction factor and the microvascular resistance correction factor both define the relation between the reference values to the respective values in the diseased state.

As such, a correction factor may particularly refer to a factor to be multiplied with a reference value to correct the reference value for disease-induced changes, such as to define the respective (affected) health parameter value.

In this context, it shall be understood that the above-mentioned changes compared to the reference value may not only be caused by specific co-morbidities, but may also be due to the patient's general health state. As an example, the archived patient data may also indicate the patient's age. The age of the patient may also be used to estimate the above-mentioned correction factors. More particularly, the age of the patient may be considered a health parameter suitable to extract the correction factor $\beta$ for the microvascular resistance. More specifically, the microvascular resistance typically increases with age. Accordingly, it may be assumed that the correction factor $\beta$ should be larger for patients of a higher age and smaller for patients that are younger.

In yet another embodiment, the extraction unit comprises a machine learning algorithm. Further, the archived patient data comprises a patient data matrix that is input into the machine learning algorithm for training the algorithm and the extracting the at least one correction factor comprises a prediction of the at least one correction factor based on said training.

In some embodiments, a machine learning algorithm is used to apply the correction to the intravascular measurement data. In this context, the term machine learning algorithm refers to a situation where a computer is trained to perform a task by means of respective sample data without being explicitly programmed. To that end, machine learning particularly refers to employing algorithms that use data-driven predictions or recognitions instead of strictly following static programming instructions. This is achieved by building a computation model on the basis of the sample data input.

The computation model may particularly receive a patient data matrix as a sample input. In this context, the term patient data matrix particularly refers to a matrix of medical data of the patient in question. The medical data may hereby particularly refer to diagnostic imaging data that has been acquired by different imaging modalities. Likewise, the medical data may relate to data providing information over the cardiac cycle, such as electrocardiographic (ECG-) or photoplethysmographic (PPG) data.

The matrix may further comprise medical data related to the patient's medical history, such as previous maladies, previous medications, long-term medications, or the like. Further, the matrix may comprise medical data concerning the patient's current and/or past treatment situation, such as current medication, previous invasive and non-invasive treatments et cetera. The matrix may also comprise personal patient information, such as age, gender, (medical) family history or the like, that may provide further information about potential, the current or the past (co-)morbidities registered for the patient.

Using a patient data matrix including the above information as sample input to the computation model allows to train the computation model and, thus, the algorithm. More particularly, by considering the various information about the patient, the computation model may be built such as to include all past, current and potential future disease-related factors for that particular patient that may cause a deviation of the health parameters from their reference values. Accordingly, on the basis of the disease-related factors, it may be determined which reference values for the health parameters used in the evaluation of the intravascular measurement may be affected by irregularities and, therefore, need to be corrected. In accordance with the further information about the patient, such as age, family history, gender, as well as the further medical information, the severity of the deviation, and, thus, the necessary magnitude of the correction may also be estimated. Using this information, the machine learning algorithm may therefore be configured to predict the correction factors necessary to correct the reference values for the health parameters for that particular patient, by replacing the reference value by a respectively corrected health parameter value that has been adjusted in accordance with the patient's additional maladies.

According to a further embodiment, the training of the algorithm further comprises an input of first, pressure-based values and second, flow-based values to the machine learning algorithm.

In some embodiments, the computation model may particularly be built using a database comprising corresponding health parameters that have been obtained for a particular patient using different measurement modalities. More particularly, the patient database may include at least one health parameter that has been acquired using at least two measurement modalities, such as pressure-based values relating to the patient's blood and flow-based values relating to the patient's blood. These corresponding health parameters allow to determine the correlation between the different measurement modalities for that particular patient. Thus, they allow the machine learning algorithm to be trained with respect these correlations. Since the relationship between different modalities is known for that patient, this arrangement allows to more accurately predict the one or more correction factors.

To that end, in some embodiments, the patient data matrix comprises one or more of historic patient data, patient-specific data that has been acquired using a medical measurement modality, patient-specific personal data and/or patient-specific treatment data.

In an even further embodiment, the extracting the at least one correction factor comprises an obtaining at least one reference health parameter and a deriving, from the archived patient data, at least one corresponding patient-specific health parameter. The extracting further comprises a comparing the reference health parameter and the patient-specific health parameter to one another to determine the at least one correction factor. According to yet another embodiment the at least one patient-specific health parameter comprises a hemodynamic parameter derived on the basis of at least one image data of the patient's vasculature.

In some embodiments, the extraction unit may be used to extract the at least one correction factor directly from the archived patient data. More particularly, the extraction unit may use medical measurement data comprised in the archived patient data to derive one or more patient-specific health parameter (and their respective values) to be compared to the corresponding reference health parameter. To that end, the extraction unit may also obtain the corresponding reference health parameter. Hereby, the extraction unit may obtain the corresponding reference health parameter (value) from a database. This database may correspond to the patient database or may be a separate storage medium for storing the reference health parameter values.

The extraction unit may then compare the patient-specific health parameter to the reference health parameter to obtain the at least one correction factor. In some embodiments the extraction unit may calculate the at least one correction factor as the ratio between the patient-specific health parameter value that has been obtained from the medical measurement data and the reference health parameter value. Accordingly, the correction factor may correspond to the factor defining the relation between the patient-specific health parameter value and the corresponding reference value.

In some illustrative embodiments, the archived patient data may particularly comprise respective diagnostic image data and the patient-specific health parameter is derived from the diagnostic image data. According to one exemplary embodiment, the diagnostic image data may be used to derive the left ventricular ejection fraction for the patient ($LVEF_{Patient}$), i.e. the fraction of the blood ejected from the left ventricle with each heartbeat. This derived value for the $LVEF_{Patient}$ may then be compared to the reference value $LVEF_{Reference} = 67\%$. If the derived $LVEF_{Patient}$ value is different than the reference value $LVEF_{Reference}$, a corresponding correction factor may be determined from the ratio between these two values.

In some further exemplary embodiments, the diagnostic image data obtained from the patient database may comprise one or more perfusion images that have been acquired, e.g. by computed tomography or positron-emission tomography. On the basis of these perfusion images, it may be possible to determine the blood flow rates per myocardial mass for the patient's vasculature ($U_{Patient}$). For these blood flow rates, reference values may also be obtained ($U_{Reference}$). In this exemplary embodiment, the derived value $U_{Patient}$ may thus be compared to the reference value $U_{Reference}$ to determine the correction factor. More particularly, by calculating the ratio $U_{Patient}/U_{Reference}$, the correction factor for this case may be determined.

Similar embodiments may be foreseen for further health parameters, as long as these health parameters may be derived from the medical measurement data and may be considered as having a corresponding reference value.

According to yet another embodiment, the at least one correction factor is derived on the basis of one or more of a patient's heart rate, a patient's rate pressure product, a blood oxygen level of the patient, a volume percentage of red blood cells for the patient, an occurrence of cardiac arrhythmia in the patient, and/or an irregularity in an aortic pressure for the patient.

In some embodiments, it is assumed that patient is in a resting state during the acquisition of the intravascular measurement data, such as during iFR acquisition. However, depending on the patient's health status, the resting state may be different for different patients. In order to account for this difference between patients a respective correction factor may be derived. More particularly, the respective correction factor may be derived by regarding the patient's heart rate (HR) and/or the patient's rate pressure product (RPP) as provided in the archived patient data.

To that end, the rate pressure product is defined as the product of the patient's heart rate and the patient's systolic blood pressure (SBP):

$$RPP = HR * SBP$$

In case the archived patient data indicates irregularities in the HR/RPP value, i.e. the archived patient data reveals that these values are abnormally high for the particular patient, it may derived that the HR/RPP value may also being higher than usual during the intravascular measurement in the resting state. Thus, the intravascular measurement data has to be corrected to take account for these higher values. More particularly, it may be derived from the increased HR/RPP value that the patient's heart is experiencing more stress even in resting conditions. This results in a higher blood demand of the heart, which, in turn, causes the resistance of the microvasculature to be reduced compared to the reference case. In the context of the above-explained exemplary embodiment, this means that the correction factor $\beta$ used to correct the microvascular resistance according to $\beta R_{M-healthy} = R_{M-diseased}$ has to be smaller than the standard value 1.0.

In some embodiments, in which the patient is in a resting state during the acquisition of the intravascular measurement data, the blood oxygen level and/or the hematocrit value, i.e. the volume percentage of the red blood cells in the blood, may be determined from the archived patient data.

More particularly, it may be determined that the blood oxygen level and/or the hematocrit value is lower than usual. In both cases, higher blood volumes are necessary to provide the same amount of oxygen. Thus, a reduction in the blood oxygen level and/or the hematocrit value results in a higher demand of the blood volume, which, in turn, reduces the microvascular resistance. Accordingly, from this information it may also be derived that the correction factor $\beta$ has to be smaller than the standard value 1.0.

In some embodiments, the patient is in a state of hyperemia during the acquisition of the intravascular measurement data, such as during FFR acquisition. In these cases, the heart rate (HR) and rate pressure product (RPP) may also be used to derive at least one respective correction factor. More particularly, if it is determined that the HR and/or RPP value is higher than usual, this may cause the aortic pressure value ($P_{a-diseased}$) to be increased compared to the reference value. This means that the correction factor $\alpha$ used to correct the aortic pressure according to $\alpha P_{a-healthy} = P_{a-diseased}$ has to be larger than the standard value 1.0.

In some embodiments, it may be derived from the archived patient data that the patient has previously suffered from cardiac arrhythmia. More specifically, ECG or PPG data may indicate these cardiac arrhythmia. This information may be used to determine that the correction factor $\alpha$ for the aortic pressure has to be smaller than the standard value 1.0, since arrhythmias typically reduce the pumping efficiency of the heart. Hereby, the magnitude of the correction factor $\alpha$ may particularly be determined based on the frequency and type of the cardiac arrhythmia.

In some embodiments, the archived patient data may reveal that the aortic pressure of the patient shows irregularities compared to a healthy state. More particularly, it may be revealed that the value of the aortic pressure is increased or reduced compared to the reference value. This information may be used as an indicator that the correction factor $\alpha$ for the aortic pressure needs to be reduced or increased, respectively, compared to the reference value.

According to a further aspect, a system comprising the apparatus for evaluating the patient's vasculature and a patient database communicatively connected to the apparatus is provided. In a further embodiment, the patient database comprises a Picture Archiving and Communication System (PACS) and/or a Hospital Information System (HIS) and/or a Radiology Information System (RIS).

In accordance with an embodiment, the patient database may particularly comprise a Picture Archiving and Communication System (PACS). A PACS relates to a system which is used for economical storage of and easy access to diagnostic image data of a patient that has been acquired by means of different imaging modalities. The diagnostic image data may hereby be stored digitally in the PACS, often alongside further information about the diagnostic image data that is included in respective (text) documents. Thus, by means of including a PACS into the patient database, a convenient storage entity for storing diagnostic image data for each patient may be provided.

Alternatively or additionally, the patient database may further comprise a Hospital Information System (HIS). A HIS particularly refers to a system for managing all information available with respect to hospital facilities, hospital finances, patient's records illustrating details about demographics, gender, age, et cetera of the patient's or the like. Therefore, the HIS may typically be provided with different system entities for different purposes. One particular part of the system may relate to an entity providing the patient records in which a patient's personal information, a patient's medical test results, such as laboratory results, and/or further information about the patient, such as historic information, may be stored. Thus, by including the HIS into the patient database, access to the patient's background information, medical history, previous treatment measures, test results or the like may be enabled.

Further, the patient database may additionally or alternatively comprise a Radiology Information System (RIS). The term RIS in this context refers to the core system for the electronic management inside the hospital departments that are concerned with (X-ray based) diagnostic imaging, i.e. the radiology departments. Hereby, the RIS is particularly used for patient registration and patient scheduling, workflow management, results distribution, patient tracking, modality management et cetera.

Accordingly, the functions of the RIS are very similar to that of the HIS, while being tailored to the special needs in X-ray imaging departments. Further, the RIS may be used to interface with the PACS. Therefore, in some embodiments, an examination session for a particular patient may be selected in the RIS. This selection may be indicated to the PACS via the interface and the PACS may be caused to provide the respective diagnostic image data for that examination session of said patient.

The PACS, the HIS and the RIS may be considered as complementing one another. In one particular embodiment, all three systems are included in the database. This allows to not only provide a multitude of complementary archived patient data to the apparatus, but also to correlate each of the archived patient data derived from the different systems to one another via the respective interfaces.

The provision of the thus correlated archived patient data may improve the evaluation of the patient's vasculature based on the intravascular measurement data significantly, in particular since it allows to derive specific conclusions about the corrections that may be necessary for the intravascular measurement data.

In a further aspect, a method for evaluating a patient's vasculature is provided. The method comprises the steps of receiving intravascular measurement data acquired in-situ for a vessel of interest in the patient's vasculature and receiving archived patient data for the patient from a patient database. Further, the method comprises applying a correction on the intravascular measurement data on the basis of archived patient data. In some embodiments, the method further comprises the steps of extracting at least one correction factor based on the archived patient data and correcting the intravascular measurement data using the at least one correction factor.

In a further aspect, a computer program for controlling an apparatus according to the invention is provided, which, when executed by a processing unit, is adapted to perform the method according to the invention. In an even further aspect, a computer-readable medium having stored thereon the computer program is provided.

It shall be understood that the apparatus for evaluating a patient's vasculature may be implemented by means of a processing unit. Hereby, the input unit, the correction unit and the extraction unit may be implemented as modules in a respective processing unit. The functionality of these modules may in particular be implemented by means of a respective algorithm. This algorithm may in particular be implemented using a machine learning algorithm implemented on said processing unit including said modules.

It shall be understood that the apparatus of claim 1, the system of claim 10, the method of claim 12, the computer program of claim 14, and the computer-readable medium of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically represents a system according to an embodiment including an apparatus for evaluating a patient's vasculature and a respective patient database comprising a plurality of database systems.

Fig. 2 schematically illustrates a system for evaluating a patient's vasculature including the apparatus and the patient database according to an embodiment.

Fig. 3 schematically represents a flow chart for a method for evaluating a patient's vasculature according to an embodiment.

Fig. 4 schematically represents a flow chart for a method for evaluating a patient's vasculature according to a further embodiment.

DETAILED DESCRIPTION OF EMBODIMENTS

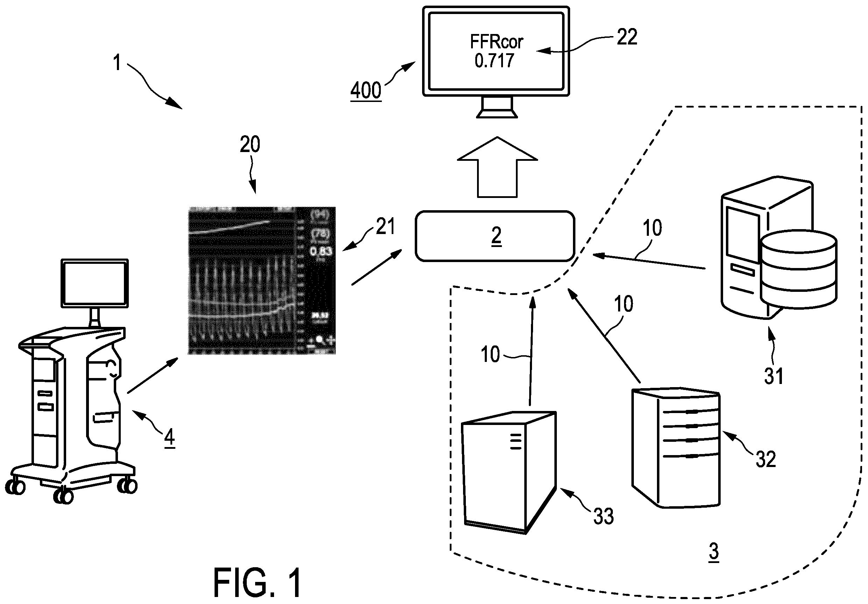

The illustration in the drawings is schematically. In different drawings, similar or identical elements are provided with the same reference numerals. Fig.1 schematically illustrates a general overview over a system 1 for evaluating a patient's vasculature according to an embodiment. The system comprises an apparatus 2 for evaluating a patient's vasculature and a respective patient database 3. Further, in the exemplary embodiment according to Fig. 1, an intravascular measurement system 4 is represented.

Intravascular measurement system 4 is used to acquire intravascular measurement data 20 for a patient. In the exemplary embodiment according to Fig. 1, intravascular measurement system 4 is used to perform an intravascular measurement of the fractional flow reserve (FFR) in the vessel of interest. Hereby, the FFR value may be considered a surrogate to indicate the remaining maximum flow capability in the vessel of interest, i.e. it is assumed that $Q_{stenosis}/Q_{healthy} \approx P_d/P_a$.

During this measurement, an FFR value 21 of 0.83 is determined. Thus, on the basis of this value, it may be derived that the remaining maximum flow capability in the vessel of interest is 83%. However, this approximation only holds true in case the factors influencing the above relation may correspond to a standard case, i.e. there are no co-morbidities that may influence this relation. Otherwise, the FFR value 21 needs to be corrected to obtain an accurate estimation of the remaining maximum flow capability.

Thus, the measured FFR value 21 is provided to apparatus 2. Apparatus 2 further receives archived patient data 10 from patient database 3. In the exemplary embodiment according to Fig. 1, the patient database comprises a Picture Archiving and Communication System (PACS) 31, a Hospital Information System (HIS) 32 and a Radiology Information System (RIS) 33. That is, the PACS 31, HIS 32 and RIS 33 together form the patient database 3. The archived patient data 10 is received from the PACS 31, the HIS 32 and the RIS 33 at the apparatus 2.

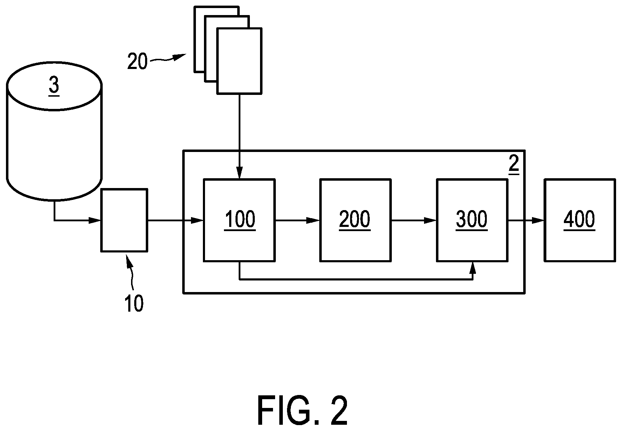

Subsequently, the apparatus 2 may determine, based on the archived patient data 10 that the patient is suffering from certain co-morbidities and that, therefore, a correction of the intravascular measurement data 20, in this case the FFR value 21, is necessary. To that end, apparatus 2 may particularly be configured to derive from the archived patient data 10 how the intravascular measurement data 20 needs to be corrected. This process is further described herein below in relation to Figs. 2 to 4. Then, apparatus 2 corrects the intravascular measurement data 20 accordingly.

In the exemplary embodiment according to Fig. 1, apparatus 2 determines a corrected FFR value 22 of 0.717 and displays this value on display unit 400. This corrected FFR value may be considered to more accurately represent the remaining maximum flow capability in the patient's vasculature. In this embodiment, the remaining maximum flow capability may thus be considered to correspond to only 71.7%, a value that necessitates a treatment of the patient contrary to the value determined for the FFR value that has not yet been corrected. Accordingly, by means of correcting the intravascular measurement value using archived patient data, more accurate conclusions may be drawn with respect the patient's vasculature.

To that end, Fig. 2 schematically illustrates a system for evaluating a patient's vasculature according to an exemplary embodiment including a more detailed view of the apparatus 2.

The system 1 again comprises an apparatus 2 and a patient database 3. The apparatus 2 comprises an input unit 100, an extraction unit 200 and a correction unit 300. The apparatus 2 is further connected to a display unit 400. The patient database 3 is communicatively connected to the apparatus 2.

Input unit 100 is configured to receive, from the patient database 3, archived patient data 10. Further, input unit 100 receives intravascular measurement data 20. Input unit 100 is configured to provide archived patient data, optionally along with information about the intravascular measurement data to extraction unit 200. Extraction unit 200 is configured to extract at least one correction factor from archived patient data 10. To this end, extraction unit 200 may optionally use the information about the intravascular measurement data 20 to determine the correction factors that need to be derived from the archived patient data 10. In the exemplary embodiment according to Fig. 2, intravascular measurement data 20 corresponds to a measurement of the fractional flow reserve (FFR) value in the vessel of interest as a surrogate to indicate the remaining maximum flow capability. However, in the exemplary embodiment according to Fig. 2, it is determined, from the archived patient data 10 retrieved from patient database 3, that the patient is suffering from a microvascular dysfunction. This microvascular dysfunction affects the aortic pressure in the patient's vasculature as well as the microvascular resistance. Thus, it is determined that the aortic pressure in the patient's vasculature has to be corrected according to

$$\alpha\, P_{a-healthy} = P_{a-diseased}.$$

Further, the microvascular resistance of the vasculature has to be corrected by

$$\beta\, R_{M-healthy} = R_{M-diseased}.$$

Considering both corrections, the state of the art approximation

$$\frac{Q_{stenosis}}{Q_{healthy}} = \frac{\frac{1}{R_M}(P_d - P_v)}{\frac{1}{R_M}(P_a - P_v)} \approx \frac{P_d}{P_a}$$

has to be corrected according to

$$\frac{Q_{stenosis}}{Q_{healthy}} = \frac{\frac{1}{\beta R_{M-healthy}}(P_d - P_v)}{\frac{1}{R_{M-healthy}}(\frac{1}{\alpha}P_{a-healthy} - P_v)} \approx \frac{P_d}{P_a}.$$

This leads to the following approximation

$$\frac{Q_{stenosis}}{Q_{healthy}} \approx \frac{\alpha \, P_d}{\beta P_a}$$

Thus, in the exemplary embodiment according to Fig. 2, extraction unit 200 may particularly be configured to derive the correction factors $\alpha$ and $\beta$ for the aortic pressure and the microvascular resistance, respectively.

To that end, in the embodiment according to Fig. 2, extraction unit 200 may particularly be implemented as a machine learning algorithm. That is, extraction unit 200 has previously been trained using a patient data matrix including diagnostic imaging data, further medical measurement data, data related to the patient's medical history, data relating to the patient's current and/or past treatment situation and data relating to the patient's personal information. Further, in the exemplary embodiment according to Fig. 2, the extraction unit, i.e. the machine learning algorithm, has been trained by providing, to the extraction unit, a database which includes a plurality of pressure based FFR-values and respective flow-based FFR values. These FFR values having been obtained on the basis of pressure and flow measurements, respectively, enable a training of the algorithm with respect to the correlation between pressure-based and flow-based measurement values.

On the basis of this training, a computation model has been built that is configured to predict correction factors $\alpha$ and $\beta$ on the basis of the archived patient data.

In the embodiment according to Fig. 2, extraction unit may therefore predict that $\alpha = 0.95$ and $\beta = 1.1$. The extraction unit may provide the predicted correction factors to correction unit 300.

Further, correction unit 300 receives an FFR value as obtained during the intravascular measurement from input unit 100 as the intravascular measurement data 20. In the exemplary embodiment according to Fig. 2, the FFR value corresponds to

$$\frac{P_d}{P_a} = 0.83.$$

According to the state of the art, this FFR value would indicate that there is still 83% remaining maximum flow capability in the vessel of interest and that a treatment is not necessary. However, due to potential co-morbidities of the patient, this value may not correctly indicate the remaining maximum flow capability.

Thus, the intravascular measurement data, i.e. the measured FFR value of 0.83, is provided to correction unit 300 to correct the value in accordance with the co-morbidities. In the embodiment according to Fig. 2, correction unit 300 then corrects the intravascular measurement data, i.e. the obtained FFR value according to

$$\frac{0.95}{1.1}0.83 = 0.717.$$

Correction unit 300 then outputs the corrected FFR value.

This output may then be displayed to a user, such as a physician, tasked with evaluating the patient's vasculature. In the exemplary embodiment according to Fig. 2, this output is provided to display unit 400, which may be a computer screen, such as a touch screen. Display unit 400 may also be implemented as a graphical user interface on a user terminal or the like. In the embodiment, the output is thus visualized for the user to acknowledge the corrected intravascular measurement data, i.e. the corrected FFR value.

To that end, the corrected FFR value indicates that there is only 71.7 % remaining maximum flow capability left in the vessel of interest. In this case, the reduction of the flow capability indeed necessitates a treatment. The user, in particular the physician, may thus derive, from the corrected measurement data, that a treatment on the patient shall be performed.

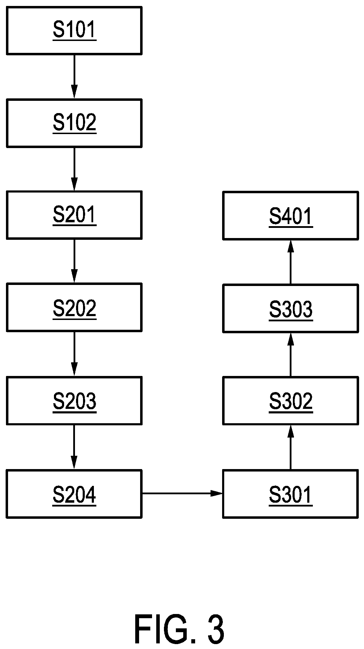

Fig. 3 schematically illustrates a flow chart for a method for evaluating a patient's vasculature according to a particular exemplary embodiment, in which the extraction unit is implemented as a machine learning algorithm.

In step S101, the intravascular measurement data 20 is received at the input unit 100. In this exemplary embodiment, the intravascular measurement corresponds to a measurement of the fractional flow reserve (FFR). Accordingly, the intravascular measurement data 20 comprises a respective FFR value.

In step S102, the archived patient data 10 is received at the input unit 100. The archived patient data 10 is provided to extraction unit 200 in step S201. In this particular embodiment, the archived patient data 10 comprises a patient data matrix as described herein above. Along with this patient data matrix, the extraction unit 200 also receives first pressure-based measurement values and second, flow-based measurement values from a database. In step S202, extraction unit is then trained as described herein above using a patient data matrix and the database including the pressure-based and flow-based measurement values. Alternatively, patient outcome data based on previous patients with similar conditions may be included in the data base. In step S203, the trained machine learning algorithm predicts the correction factors $\alpha$ and $\beta$ for the FFR measurement and provides them to correction unit 300 in step S204.

In step S301, the correction factors are received at correction unit 300. Further, the intravascular measurement data 20, in this exemplary embodiment the FFR value, is also received at correction unit 300 from input unit 100. In step S302, the correction unit 300 uses the correction factors to correct the intravascular measurement data 20, i.e. the FFR value as described herein above. In step S303, correction unit 300 outputs the corrected intravascular measurement data to display unit 400.

In step S401, display unit 400 displays the corrected intravascular measurement data, in this particular case the corrected FFR value for the vessel of interest, to a user, which may particularly be a physician. The physician may consider the corrected FFR value to be an accurate surrogate for the remaining maximum flow capability in the vasculature.

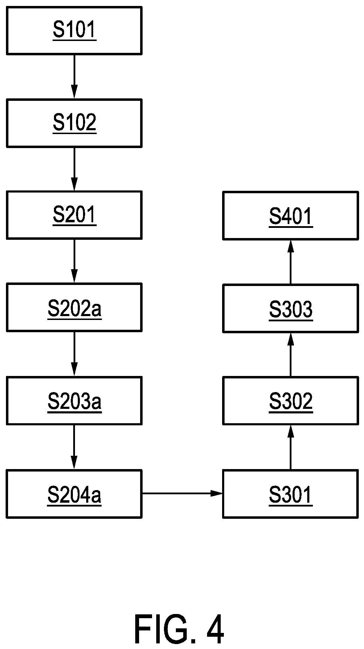

Fig. 4 further schematically represents a flow chart for a method for evaluating a patient's vasculature according to yet another embodiment, in which the correction factors may be directly derived from the archived patient data 10 by the extraction unit 200. In this exemplary embodiment, the intravascular measurement data 20 again corresponds to a measurement of an FFR value.

To that end, steps S101, S102 and S201 of the embodiment in Fig. 4 correspond to steps S101 and S102 of the embodiment according to Fig. 3. That is, in step S101, the intravascular measurement data 20 is received at the input unit 100 and, in step S102, the archived patient data 10 is received at the input unit 100. Further, in step S201, the archived patient data 10 is provided to extraction unit 200.

In step S202a, extraction unit 200 then obtains at least one reference health parameter. In the particular embodiment according to Fig. 4, extraction unit 200 may particularly obtain a reference value for the aortic pressure ($P_{a-healty}$) and the microvascular resistance ($R_{M-healthy}$), respectively. Extraction unit 200 may retrieve these reference health parameters from a reference database or, alternatively, from the patient database 3. Likewise, the reference health parameters may be retrieved from an internal storage in the apparatus.

In step S203a, the extraction unit 200 further derives, from the archived patient data 10, the corresponding health parameter for the patient whose vasculature is to be evaluated. In the specific embodiment according to Fig. 4, extraction unit 200 may particularly derive that the patient is suffering from a microvascular dysfunction. Thus, the extraction unit may determine that the aortic pressure and the microvascular resistance deviate from the reference values. Accordingly, the extraction unit 200 may derive, in step S203a, the patient-specific aortic pressure value for the patient suffering from microvascular dysfunction ($P_{a-diseased}$) and the respective microvascular resistance value ($R_{M-diseased}$).

In step S204a, the extraction unit 200 compares the values $R_{M-diseased}$ and $R_{M-healthy}$ and $P_{a-diseased}$ and $P_{a-healthy}$, respectively, to determine a correction factor $\alpha$ for the aortic pressure and a correction factor $\beta$ for the microvascular resistance.

In the embodiment according to Fig. 4, extraction unit 200 particularly calculates, in step S204a, the correction factors $\alpha$ and $\beta$ according to

$$\alpha = \frac{P_{a-diseased}}{P_{a-healthy}}, \beta = \frac{R_{M-diseased}}{R_{M-healthy}}$$

and provides them to correction unit 300.

The following steps then correspond to the method steps described in relation to the embodiment according to Fig. 4. That is, the thus determined correction factors are then received at correction unit 300 in step S301.

Further, the intravascular measurement data, in this exemplary embodiment the FFR value, is also received at correction unit 300 from input unit 100. The correction unit 300 uses the correction factors in step S302 to correct the intravascular measurement data and outputs, in step S303, the corrected intravascular measurement data to display unit 400.

Subsequently, in step S401, display unit 400 displays the corrected intravascular measurement data. In this particular embodiment, the corrected intravascular measurement data, which corresponds to a corrected FFR value, is displayed to a physician. The physician may then consider potential treatment options on the basis of the corrected value, which may be considered an accurate indication of the remaining maximum flow capabilities.

Although in above described embodiments, the patient database has been defined as comprising a PACS, a HIS and a RIS, it shall be understood that in other embodiments, the patient database may likewise comprise other and/or additional systems, such as a Nursing Information System (NIS), a Physician Information System (PIS) and/or a Pharmacy Information System or the like.

Further, it shall be understood that, although in the above embodiments the health parameters that have been regarded have been related to the aortic pressure, the microvascular resistance, the ejection fraction and/or the blood flow rates, any other health parameters may also be used, as long as they influence the intravascular measurements and clinically normal averages may be determined from them.

Further, while in the above embodiments, the evaluation has been performed on the coronary vasculature, in other embodiments, the evaluation may likewise be performed on the vasculature in other parts of the human body, such as the peripheral vasculature.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the receiving of the intravascular measurement data and the archived patient data, the extracting of the correction factor, the correcting the intravascular measurement data, the comparing of health parameters, et cetera performed by one or several units or devices can be performed by any other number of units or devices. These procedures in accordance with the invention can hereby be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for evaluating a patient's vasculature, comprising an input unit configured to receive intravascular measurement data acquired in-situ for a vessel of interest in the patient's vasculature and archived patient data for the patient from a patient database. The apparatus further comprises a correction unit configured to apply a correction to the intravascular measurement data on the basis of archived patient data.

By means of this apparatus, it is possible to correct intravascular measurement data for irregularities caused by patient specific health issues and co-morbidities, thereby avoiding incorrect assessment of the patient's vasculature due to factors that have not been considered previously.

**Claims**

1. An apparatus for evaluating a patient's vasculature, comprising
   an input unit configured to receive

   intravascular measurement data acquired in-situ for a vessel of interest in the patient's vasculature; and
   archived patient data for the patient from a patient database;

   a correction unit configured to apply a correction to the intravascular measurement data on the basis of archived patient data.

2. Apparatus according to claim 1, wherein the intravascular measurement data is acquired by a measurement of a Fractional Flow Reserve (FFR) and/or an Instantaneous wave-Free Ratio (iFR).

3. Apparatus according to claim 1, further comprising an extraction unit configured to extract at least one correction factor based on the archived patient data; wherein
   the correction unit is configured to apply the at least one correction factor to the intravascular measurement data.

4. Apparatus according to claim 3, wherein the at least one correction factor is related to an aortic pressure value and/or a resistance of a microvasculature of the patient.

5. Apparatus according to claim 3, wherein the extraction unit comprises a machine learning algorithm;
   the archived patient data comprises a patient data matrix that is input into the machine learning algorithm for training the algorithm; and
   the extracting the at least one correction factor comprises a prediction of the at least one correction factor based on said training.

6. Apparatus according to claim 5, wherein the training of the algorithm further comprises an input of first, pressure-based values and second, flow-based values to the machine learning algorithm.

7. Apparatus according to claim 5, wherein the patient data matrix comprises one or more of historic patient data, patient-specific data that has been acquired using a medical measurement modality, patient-specific personal data and/or patient-specific treatment data.

8. Apparatus according to claim 3, wherein the extracting the at least one correction factor comprises
   obtaining at least one reference health parameter;
   deriving, from the archived patient data, at least one corresponding patient-specific health parameter; and
   comparing the reference health parameter and the patient-specific health parameter to one another to determine the at least one correction factor.

9. Apparatus according to claim 8, wherein

the at least one patient-specific health parameter comprises a hemodynamic parameter derived on the basis of at least one image data of the patient's vasculature.

10. A system for evaluating a patient's vasculature, the system comprising:

an apparatus for evaluating a patient's vasculature according to anyone of claims 1 to 9; and a patient database communicatively connected to the apparatus.

11. System according to claim 10, wherein the patient database comprises a Picture Archiving and Communication System (PACS) and/or a Hospital Information System (HIS) and/or a Radiology Information System (RIS).

12. Method for evaluating a patient's vasculature, the method comprising the steps of:

receiving intravascular measurement data acquired in-situ for a vessel of interest in the patient's vasculature; and receiving archived patient data for the patient from a patient database; and applying a correction to the intravascular measurement data on the basis of archived patient data.

13. Method according to claim 12, further comprising the steps of extracting at least one correction factor based on the archived patient data; and applying the at least one correction factor on the intravascular measurement data.

14. A computer program for controlling an apparatus according to anyone of claims 1 to 9, which, when executed by a processing unit, is adapted to perform the method according to anyone of claims 12 or 13.

15. A computer-readable medium having stored thereon the computer program according to claim 14.

FIG. 1

EP 3 456 249 A1

FIG. 2

FIG. 3

FIG. 4

Europäisches
Patentamt

European
Patent Office

Office européen
des brevets

# EUROPEAN SEARCH REPORT

Application Number

EP 17 19 1426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/039276 A1 (HATTANGADI NEIL [US] ET AL) 6 February 2014 (2014-02-06)<br>* paragraph [0026] - paragraph [0034] *<br>* paragraph [0053] - paragraph [0061] *<br>* figures 1,2,7 *<br>* paragraph [0003] *<br>----- | 1-15 | INV.<br>A61B5/02<br>G16H30/20<br>G16H10/60<br><br>ADD.<br>A61B5/0215<br>A61B5/00 |
| X | WO 2011/148340 A1 (UNIV RAMOT [IL]; ROTMAN OREN [IL]; ARONIS ZEEV [IL]; ROSEN LIOR [IL];) 1 December 2011 (2011-12-01)<br>* page 9, line 25 - page 10, line 8 *<br>* page 12, line 32 - page 13, line 15 *<br>* page 30, line 18 - line 32 *<br>----- | 1,3,10, 12-15 | |
| X | WO 2017/041368 A1 (SUZHOU RUNXIN MEDICAL TECH CO LTD [CN]) 16 March 2017 (2017-03-16)<br>* page 7, paragraph 7 - last paragraph *<br>* page 14, paragraph 8 - page 15, paragraph 3 *<br>* figures 2-4 *<br>----- | 1,2,10, 12,14,15 | |
| X | WO 2016/094094 A1 (MEDTRONIC VASCULAR INC [US]) 16 June 2016 (2016-06-16)<br>* paragraph [0028] - paragraph [0035] *<br>* figures 4,5 *<br>----- | 1,12,14, 15 | TECHNICAL FIELDS SEARCHED (IPC)<br><br>A61B<br>G16H |
| A | US 2014/180032 A1 (MILLETT BRET C [US] ET AL) 26 June 2014 (2014-06-26)<br>* paragraph [0032] - paragraph [0035] *<br>* paragraph [0040] *<br>* paragraph [0091] - paragraph [0092] *<br>* paragraph [0108] *<br>* figures 1A,9 *<br>----- <br>-/-- | 1-15 | |

The present search report has been drawn up for all claims

| Place of search | Date of completion of the search | Examiner |
|---|---|---|
| The Hague | 7 March 2018 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 17 19 1426

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/112182 A1 (SHARMA PUNEET [US] ET AL) 23 April 2015 (2015-04-23)<br>* paragraph [0027] - paragraph [0029] *<br>* paragraph [0035] - paragraph [0036] *<br>* figures 1,2 *<br>----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 7 March 2018 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 1426

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2018

| Patent document cited in search report | | Publication date | Patent family member(s) | | Publication date |
|---|---|---|---|---|---|
| US 2014039276 | A1 | 06-02-2014 | CA | 2880747 A1 | 06-02-2014 |
| | | | EP | 2879573 A1 | 10-06-2015 |
| | | | EP | 3184048 A1 | 28-06-2017 |
| | | | EP | 3298959 A2 | 28-03-2018 |
| | | | JP | 6181180 B2 | 16-08-2017 |
| | | | JP | 2015527136 A | 17-09-2015 |
| | | | US | 2014039276 A1 | 06-02-2014 |
| | | | WO | 2014022804 A1 | 06-02-2014 |
| WO 2011148340 | A1 | 01-12-2011 | NONE | | |
| WO 2017041368 | A1 | 16-03-2017 | CN | 105078425 A | 25-11-2015 |
| | | | WO | 2017041368 A1 | 16-03-2017 |
| WO 2016094094 | A1 | 16-06-2016 | EP | 3229675 A1 | 18-10-2017 |
| | | | US | 2016166158 A1 | 16-06-2016 |
| | | | WO | 2016094094 A1 | 16-06-2016 |
| US 2014180032 | A1 | 26-06-2014 | CA | 2895815 A1 | 26-06-2014 |
| | | | EP | 2934305 A1 | 28-10-2015 |
| | | | JP | 2016508043 A | 17-03-2016 |
| | | | US | 2014180032 A1 | 26-06-2014 |
| | | | WO | 2014099935 A1 | 26-06-2014 |
| US 2015112182 | A1 | 23-04-2015 | CN | 106456078 A | 22-02-2017 |
| | | | EP | 3057510 A1 | 24-08-2016 |
| | | | US | 2015112182 A1 | 23-04-2015 |
| | | | US | 2016106321 A1 | 21-04-2016 |
| | | | US | 2017265754 A1 | 21-09-2017 |
| | | | WO | 2015058044 A1 | 23-04-2015 |

EPO FORM P0459

For more details about this annex : see Official Journal of the European Patent Office, No. 12/82